# EUROPEAN PATENT APPLICATION

(11) **EP 4 410 212 A1**
(43) Date of publication of application: **07.08.2024**
(21) Application number: 23305131.7
(22) Date of filing: 02.02.2023
(51) Int. Cl.: A61B 6/00, G06T 7/00, G06T 7/10, G16H 50/00

(54) **VASCULAR DIAGNOSTIC APPARATUS AND METHOD**

(71) Applicant: Koninklijke Philips N.V., 5656 AG Eindhoven (NL)
(72) Inventor: FLORENT, Raoul, 5656 EINDHOVEN (NL); LEVRIER, Claire, 5656 EINDHOVEN (NL); VAN DER HORST, Arjen, 5656 EINDHOVEN (NL); THIS, Alexandre, 5656 EINDHOVEN (NL)
(74) Representative: Philips Intellectual Property & Standards

(57) **Abstract**

Presented are concepts for vascular diagnosis that comprise generating a composite view of a field of view of the patients anatomy, the composite view comprising diagnostic data. More particularly, the method includes obtaining angiogram data of a patient's vascular system, processing the angiogram data to generate a semantic angiogram data; processing the semantic angiogram data to determine one or more velocity curves according to one or more locations; determining one or more characteristic from the one or more velocity curves according to the one or more locations; generating diagnostic data corresponding to the one or more locations; and generating a composite view comprising the diagnostic data.

## Description

### FIELD OF THE INVENTION

The present invention relates to vascular diagnostic apparatus and methods.

### BACKGROUND OF THE INVENTION

Vascular diagnostic apparatus are used to diagnose vascular health. Prior approaches have utilized some flow audible characteristics as diagnostic material in vascular disease.

Non-invasive spectral Doppler waveform analysis has been used for decades as one of the main diagnostic tools for vascular diseases. Current apparatus and methods for vascular diagnosis employ duplex doppler ultrasound. Doppler ultrasound allows visual blood flow waveforms to be produced, which allows more quantitative and broader assessments to be produced.

Ultrasound suffers nevertheless from strong limitations. Some vessels are difficult to be correctly visualized in ultrasound, in particular when they are too deep within the body or too close to bones (for example, the popliteal area).

Furthermore, in the context of endovascular interventional X-ray, ultrasound usage requires the presence of a sonographer in the cathlab, which might be problematic for several reasons, such as lack of skilled person availability, disruption of the workflow, and reluctance of sonographers to practice in an ionised environment.

### SUMMARY OF THE INVENTION

The invention is defined by the claims.

According to examples in accordance with an aspect of the invention, there is provided a vascular diagnosis system, comprising at least one processor, the at least one processor configured to: obtain angiogram data of a patient's vascular system, said angiogram data comprising a field of view of a patient's vascular system; process the angiogram data to generate a semantic angiogram data comprising information on the presence and position of anatomical features present in the field of view; process the semantic angiogram data to determine one or more velocity curves according to one or more locations, wherein a velocity curve comprises data of blood velocity against time for a specific location in the field of view; determine one or more characteristics from the one or more velocity curves according to the one or more locations; generate diagnostic data corresponding to the one or more locations, from the one or more characteristics and the semantic angiogram data; and generate a composite view comprising the diagnostic data.

Proposed embodiments enable the generation of a composite view showing diagnostic data according to one or more positions in the field of view. The diagnostic data may include the data or characteristics corresponding to a plurality of velocity curves that represent different positions in the field of view. Further, the diagnostic data may comprise a diagnostic indication of a condition present within the field of view. The diagnostic data may be generated in consideration of the semantic angiogram data which provides semantic information on the positions and nature of the vessels that may be present within the field of view, additionally in consideration of one or more diagnostic rules to specific locations within the field of view.

The resultant composite view provides an automatic vascular assessment across the full X-ray field of view, overcoming many of the drawbacks of the prior art.

The approach of the current invention mitigates some or all of the mentioned problems related to the use of ultrasound, such as sonographer availability, issues with deep vessels, or workflow constraints.

According to the proposed concepts, measurement is simpler as not only does the prior art ultrasound require reasonably accessible vessels, but it also requires that the ultrasound beam is aligned with the vessel. If alignment is not ensured, the velocity is then not correctly measured. This is not an issue for X-Ray based measurement techniques.

Contrary to what is currently achievable with ultrasound, the proposed concepts do not analyse only one spatial location (say a given vessel segment), but all the sizable vessels within the field of view. Accordingly, the composite view generated according to the invention allows the provision of a more complete and more useful picture to the user.

The concepts disclosed do not require any manual interaction to generate the diagnostic data across the field of view. The invention therefore is much faster than any known diagnostic technique.

The invention enables the direct comparisons of vessels with surrounding vessels, and can use these comparisons to derive additional diagnostic descriptors (such as velocity peak ratios).

The invention offers the ability to integrate local diagnostic elements to produce a fully-fledged diagnostic map throughout the field of view.

In embodiments, the diagnostic data corresponding to the one or more locations is generated by applying one or more diagnostic rules at each of the specific locations of the one or more locations, wherein each of the one or more diagnostic rules comprises: comparing the one or more characteristic to one or more reference characteristic; and comparing the specific location to one or more reference locations, to determine a diagnostic indication of whether a condition is present at the specific location.

Accordingly, one or more diagnostic rules can be applied to specific locations within the field of view to determine if that location and characteristic at that location correspond respectively to a reference location and a reference characteristic. If the location corresponds to a reference location, and the characteristic corresponds to a reference characteristic, it may thus be determined that a condition is likely to be present at a specific location within the field of view. In embodiments, the diagnostic indication may have a confidence value associated with it, indicating how confident the system is that that diagnostic indication is present. Such diagnostic rules are known in the art.

In embodiments, the comparing the one or more characteristic to one or more reference characteristic comprises generating a characteristic value based on the characteristic at the specific location and comparing the characteristic value to one or more reference characteristic value or range.

For example, a blood flow value at a position may be determined and compared to a reference blood flow value or blood flow value range, to determine if a condition is present at that specific position within the field of view.

In embodiments, the processor is configured to obtain one or more further patient characteristic, and wherein the diagnostic rule further comprises comparing the further patient characteristic to one or more further reference characteristic.

For example, the characteristic may comprise an aspect of the medical history or medical status of the patient, which may affect the determination of whether a condition is present at a particular position when considering the characteristic at that location to a reference characteristic. For example, if a patient is on a particular medication, or has a family history indicating a predisposition to a particular condition, this may affect the expected characteristics at one or more locations, and the diagnostic indication (i.e., the determination of whether a condition is present at a particular location) may be adjusted accordingly.

In embodiments, the one or more characteristic comprises a degree of stenosis at a specific location.

In embodiments, the generating of diagnostic data corresponding to the one or more locations comprises evaluating one or more characteristic corresponding to a different location to the specific location.

Accordingly, for each identified vessel, a list of "similar" surrounding vessels can be observed or detected or known in advance and identified. These surrounding vessels can be used as comparison points to sustain a given diagnostic.

Unlike the prior approaches, in the present invention diagnostic data is generated for multipole points in the field of view simultaneously, thus a more accurate diagnosis can be provided by evaluating the characteristic(s) at other locations rather than just in a subject location.

In embodiments, the one or more of the one or more diagnostic rules is modifiable or selectable by a user resulting in a modified diagnostic indication of whether a condition is present at the specific location.

Accordingly, the preferences and experience of user (operator) can be factored into the diagnostic indication.

In embodiments, the at least one processor is configured to generate: a vessel segmentation map comprising information on which portions of the field of view comprise vessels; and one or more labels, comprising an identification of a specific vessel present the field of view and an associated position of the specific vessel; enabling the extraction of at least one semantic vessel from the vessel segmentation map, enabling the processing of the angiogram data to generate a semantic angiogram data comprising information on the presence and position of anatomical features present in the field of view.

This may provide a convenient method to generate the semantic angiogram data.

Furthermore, vessels may be semantically segmented, the invention enables the indexing within a diagnostic rule table. This would not be possible if the vessels are not semantically segmented.

In embodiments, the characteristic comprises one or more of a flow direction, a phasicity, or a curve characteristic.

The determination of one or more characteristics or descriptors can be critical in vascular diagnosis.

Flow direction comprises a determination of whether velocity is oriented from the proximal to the distal part of the vessel.

Phasicity relates to a phasic characteristic of the velocity curves. Velocity curves are usually characterised as monophasic, biphasic or triphasic. The phasicity can be deduced by measuring the behaviour of the curve with respect to zero-velocity axis crossing.

Monophasic qualifies a blood flow or velocity that remains positive, never substantially crossing the zero-velocity axis during the cardiac cycle. In the biphasic case, two periods can be observed during the cardiac cycle: a positive velocity period and a negative velocity one. In the triphasic cases, two positive-velocity and one negative-velocity periods constitute the structure of the flow curve along the cardiac cycle.

Pulsatility refers to blood flow or velocity variations along the cardiac cycle. It can for instance be expressed as the ratio of the difference between the maximal and minimal velocity observed at a given vessel location along the cardiac cycle to the average velocity observed in the same condition (same location, same temporal interval). The higher pulsatility is, the higher blood velocity varies along the cardiac cycle. The lower pulsatility is, the closer to a constant value function the velocity curve looks like. A zero-value pulsatility indicates a velocity profile that is absolutely constant along the cardiac cycle.

Curve characteristic means any other characteristic of the curve, which may include shape, amplitude, slope, or any other characteristic estimated globally on the curve or locally, for instance during a specific section of the cardiac cycle. For example, the characteristic may include a characteristic of the systolic phase (top portion of the curve), of a characteristic of the diastolic phase (bottom portion of the curve), of a comparison of a feature of the systolic and diastolic phase, or of a gap between either of the systolic and diastolic phases.

In embodiments, the curve characteristic comprises one or more of a blood flow resistance indication or an upstroke characteristic.

A blood flow resistance indication is a feature of the velocity curve that indicates the presence of a blood flow resistance somewhere in the patient's body within the connected vasculature. The resistance may cause reduced blood flow within a specific vessel in the field of view. The cause of the blood flow resistance may be outside of the field of view, for instance in the arteriola or capillaries. The blood flow resistance may be a restriction causing increased resistance to blood flow and thus resulting in lower blood flow compared to expected blood flow. A distal resistance impacts the full upstream connected vasculature.

The resistance indication may comprise: a high-resistive curve (identified by a sharp systolic upstroke, brisk downstroke); a low-resistive curve (identified by a prolonged downstroke in late systole with continuous forward flow throughout diastole without an end-of-systole notch), which is monophasic; or an intermediate-resistive curve (identified by mixed high and low-resistive characteristics).

The upstroke is the curve feature associated with the beginning of the systolic phase. The upstroke characteristic may comprise a rate of rise, for example, a rapid rise, or a prolonged rise. In the case of foot analysis, this upstroke may be referred to as Pedal Acceleration Time, which may be a good indicator to evaluate lower limb arterial perfusion.

In embodiments, the at least one processor is configured to process the angiogram data to compensate for a motion present when the angiogram data is generated, resulting in the generation of motion-compensated semantic angiogram data, and optionally wherein the motion present comprises one or more of: a patient movement; an organ movement; and a table movement.

Accordingly, motion artefacts may be reduced and a higher quality composite view may be produced.

Thus, the most common causes of motion artefacts may be overcome.

Since temporal analysis involved in velocity curve estimation requires neat removal of background elements, motion compensation is applied to the input angiogram to cancel a possible patient, organ, or table movement, a combination of these. Rigid or elastic motion compensations can be considered. Computer vision or learning techniques are possible approaches, together with their combination. The resulting vector field is used to register angiogram and semantic data. This produces a Semantic Digital Subtraction Angiogram where all the data are temporally aligned, and where background is suppressed.

In embodiments, the system further comprises a user interface, and wherein the at least one processor is configured to display the composite view on the display, and optionally wherein the user interface is configured to receive an input to allow the user to modify or manipulate the composite view on the user interface.

Displaying the composite view allows a clinician to review the composite view and support the clinician to determine a diagnosis.

Accordingly, the final diagnostic overview, in the form of a composite view, can be further tailored by the user depending on how he valued observed characteristics. Tailored deviations to guide-lines can thus be introduced. Likewise, the rule database used to produce a diagnostic can be modified according to local preferences or belief.

In embodiments, the one or more characteristic comprises the pulsatility of blood flow and the composite view comprises a blood pulsatility map corresponding to the field of view.

The blood pulsatility map shows the blood pulsatility at a plurality of positions within the field of view.

In an embodiment, the one or more characteristic comprises the phasicity of blood flow and the composite view comprises a blood phasicity map corresponding to the field of view. In an embodiment the one or more characteristic comprises the pulsatility of blood flow and the phasicity of blood flow and the composite view comprises a pulsatility map and/or a blood phasicity map of the field of view. In an embodiment, the composite view can be adjusted between a blood phasicity map and a blood pulsatility map and vice versa.

According to another aspect, there is provided a computer-implemented method of vascular diagnosis, comprising the steps of obtaining angiogram data of a patient's vascular system, said angiogram data comprising a field of view of a patient's vascular system; processing the angiogram data to generate a semantic angiogram data comprising information on the presence and position of anatomical features present in the field of view; processing the semantic angiogram data to determine one or more velocity curves according to one or more locations, wherein a velocity curve comprises data of blood velocity against time for a specific location in the field of view; determining one or more characteristic from the one or more velocity curves according to the one or more locations; generating diagnostic data corresponding to the one or more locations, from the one or more characteristics and the semantic angiogram data; and generating a composite view comprising the diagnostic data.

It should be understood that the present invention provides a method to generate vascular diagnosis imagery and/or data that may be used for vascular diagnosis. Thus, according to another aspect, there is provided a computer-implemented method of generating vascular diagnosis imagery and/or data, comprising the steps of: obtaining angiogram data of a patient's vascular system, said angiogram data comprising a field of view of a patient's vascular system; processing the angiogram data to generate a semantic angiogram data comprising information on the presence and position of anatomical features present in the field of view; processing the semantic angiogram data to determine one or more velocity curves according to one or more locations, wherein a velocity curve comprises data of blood velocity against time for a specific location in the field of view; determining one or more characteristic from the one or more velocity curves according to the one or more locations; generating diagnostic data corresponding to the one or more locations, from the one or more characteristics and the semantic angiogram data; and generating a composite view comprising the diagnostic data.

According to another aspect, there is provided a computer program product comprising computer program code means which, when executed on a computing device having a processing system, cause the processing system to perform all of the steps of the methods disclosed herein.

In embodiments, two or more sets of angiogram data of a patient's vascular system are acquired, wherein the first set is collected at a first x-ray angle with respect to the patient and at least the second set is collected at a second, different, x-ray angle with respect to the patient. In a further embodiment, the angle difference between the first x-ray angle and the at least second x-ray angle is 30 degrees or more. The angle may be measured with respect to a centre of mass of a patient or a central position within the body or the part of the anatomy that is being examined, or more traditionally, in the frame of reference of the imaging system plus table.

Accordingly, depth may be more accurately represented. X-Ray, as a projection imaging modality, may suffer from foreshortening (i.e., a discrepancy between real and projected lengths). This has a direct impact on velocity measurement. In many situations, vessels are well aligned with the projection plane, and very little foreshortening remains. However, one option to overcome potential foreshortening is to use two angiograms taken under different angulations (typically more than 30 degrees apart) and to reconstruct depth from those two angulations. For the velocity curve determination for each vessel, the projection with the least amount of foreshortening is taken. This is sometimes called 3D vessel modelling. Two or more adjacent fields of view can be stitched together to create a larger field of view. This can be done with both the anatomical map (semantic angiogram data) and with velocity curves relating to the same or similar time period. The actual distances along the vessel path can then be measured on this 3D vessel modelling, and foreshortening can be corrected accordingly. But when resorting to several differently angulated angiograms, one can also estimate the velocity curves from those several angiograms. It is then possible to combine or select the velocity curves or derived characteristics from those different angiograms. For instance, one might select at a given location of the map, the velocity curve or related characteristics corresponding to the angiogram with the least amount of foreshortening. Likewise, when different angiograms are available that together span a wider field of view in an adjacent manner, the corresponding velocity curves or related characteristics can be combined to create a diagnostic composite view over this extended field of view.

Several angiograms can also be considered in the case of an endovascular intervention, where at least one of these angiograms corresponds to a time interval preceding the intervention, which can typically be a ballooning or stenting procedure, and where at least one of these angiograms corresponds to a time interval following the intervention. In this situation, a diagnostic composite view might be created from the characteristics extracted from the velocity curves of the at least two angiograms. In this situation, the diagnostic composite view might be differential in the sense that it will reflects diagnostic assessment differences dependent on the effect of the intervention. For instance, it might inform to which degree pulsatility or flow has been restored after the deployment of a balloon or a stent at the considered location.

In embodiments, the system generates angiogram data, and associated semantic angiogram data, relating to the same field of view at two different time periods. The time periods may be one or more days apart, for example, first semantic angiogram data may be generated in a first time period and second angiogram data may be generated in a second time period, which may, for example, be one week or one month later. Accordingly, a clinician may then, as an example, compare a pair of angiograms of the same vascular field before and after treatment, to monitor how a condition may have changed in the elapsed period between the first and second time period. In an embodiment, the diagnostic view can be adjusted to show imagery relating to different time periods.

In an embodiment, a plurality of fields of views are combined together to create a larger composite view.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 is a simplified block diagram of a proposed embodiment 10;
Figure 2 shows a plurality of velocity curves;
Figure 3 shows a comparison view of a velocity curve from a first position and a second position;
Figure 4 is a simplified flow diagram of a proposed vascular diagnosis method 40;
Figure 5 illustrates an example of a computer 50 within which one or more parts of an embodiment may be employed; and
Figure 6 illustrates a possible diagnostic view 61 alongside unprocessed angiogram imagery 60.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality.

It should be understood that the Figures are merely schematic and are not drawn to scale. It should also be understood that the same reference numerals are used throughout the Figures to indicate the same or similar parts.

By way of example only, illustrative embodiments may be utilized in many different types of clinical, medical or subject-related environments, such as a hospital, doctor's office, medical-research facility, ward, care home, person's home, etc.

The invention provides methods and systems for generating a composite diagnostic view for aiding a clinician. The composite view comprises anatomical information and diagnostic data. In particular the generated diagnostic view may comprise a presentation of diagnostic data according to multiple locations within the field of view, which may enable the clinician to make an improved diagnosis or assessment. The diagnostic view may also provide one or more diagnostic indication of whether a condition is present at a particular location within the field of view, i.e., the view may indicate both the presence and location of such a condition.

Diagnostic data from adjacent vessels may be used to generate the diagnostic view. This allows a more comprehensive and complete diagnosis to be performed.

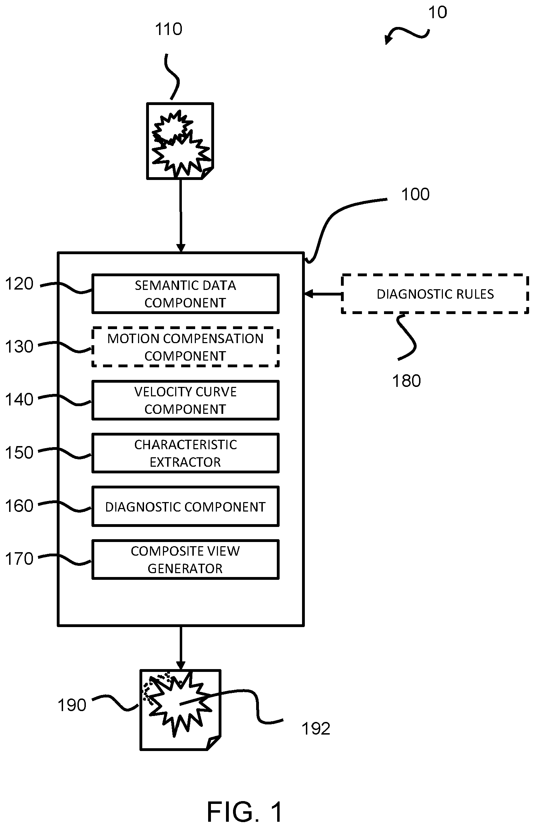

The diagnostic data for each location may comprise raw diagnostic data such as data directly derivable from a velocity curve for each location, or may comprise a diagnostic indication according to this data. For example, it may be known that in a particular vessel, a particular characteristic may be indicative of a particular condition. Accordingly, comparing a characteristic from the raw diagnostic data from a plurality of locations, in consideration of the location in question, a diagnostic indication may be derived. For example, if the location matches a reference vessel and the characteristic matches a value or falls within a reference range, a diagnostic indication may be generated, to indicate that a condition appears to be present within the field of view. Optionally the composite view may indicate the characteristic that has led to this diagnosis, and may further indicate the location at which that characteristic was observed. The location indication may be visual with respect to the field of view and/or a descriptor of the related vessel at where the condition has been observed.
To aid understanding of the proposed concept(s), an exemplary embodiment of a vascular diagnosis system will now be described with reference to Figure 1.

Figure 1 is a simplified block diagram of a proposed embodiment 10. A system 100 according to a proposed embodiment is configured to obtain (e.g., receive via an input interface) angiogram data 110 of a patient. The angiogram data comprises a field of view of a patient, thus covering one or more parts of the anatomy or of a patient's body.

The generation of angiogram data is well known in the art and a detailed description is therefore omitted.

The system 100 comprises a microcontroller and comprises several functional components. The system comprises: a semantic data component 120; a motion compensation component 130; a velocity curve component 140; a characteristic extractor 150; a diagnostic component 160; a composite view generator 170. One or more of the functional components may be combined. The system receives as, an optional input, one or more diagnostic rules 180.

The semantic data component 120 generates semantic angiogram data comprising information on the presence and position of anatomical and biological features present in the field of view. The semantic data component 120 analyses the angiogram data 110 to determine the vessels and nature of the vessels present in the field of view. As an example, the semantic data component 120 generates digital subtract angiography (DSA) data from the angiogram data 110.

As an example, the semantic data component 120 generates a vessel segmentation map comprising information on which portions of the field of view comprise vessels and one or more labels, each label comprising an identification of a specific vessel present the field of view and an associated position of the specific vessel. This information enables the extraction of at least one semantic vessel from the vessel segmentation map, thus enabling the processing of the angiogram data to generate a semantic angiogram data comprising information on the presence and position of anatomical features present in the field of view.

Given a targeted vascular field (for example, peripheral, renal, carotid, neuro...), deep learning technologies can produce vessel segmentation maps which, at each pixel of an angiogram of the targeted region, produces a vesselness probability. In addition, a label or several labels can be associated with the segmented pixels that indicate(s) which vessel(s) this pixel belongs to. A U-Net (a convolutional neural network that was developed for biomedical image segmentation) sufficiently trained is a typical example of such a realisation. Finally, the segmentation network is equally trained to detect and quantify stenoses along the analysed vessels.

The segmentation and label maps are dependable but do not provide a high-level representation. As an improvement, computer vision techniques relying for instance on minimal path techniques fed by those maps can be involved to provide the desired high-level view as a set of labelled centerlines and borderlines for each identified vessel segment. Stenosis presence and severity can also adorn the vessel centre lines as extra information.

The motion compensation component 130 is optional and generates motion-compensated semantic angiogram data by compensating for one or more of the patient movement, an organ movement and a table movement.

Because temporal analysis requires neat removal of background elements, motion compensation is applied to the input angiogram to cancel possible patient, organ, or table movements. Rigid or elastic motion compensations can be considered. Computer vision or learning techniques, or a combination of the two are alternative possible approaches. The resulting vector field is used to register angiogram and semantic data. This produces a Semantic Digital Subtraction Angiogram where all the data are temporally aligned, and where background is suppressed, thereby providing motion-compensated angiogram data.

Motion compensation may be performed at any suitable stage, for example prior to, or subsequent to, the generation of semantic angiogram data.

The velocity curve component 140 generates velocity curves for one or more locations in the field of view. Typically, component generates at least one velocity curve per blood vessel but may comprise generating more than one velocity curve for a vessel. Each velocity curve comprises data of blood velocity against time for a particular location. As an example, one method that can be used for the generation of a plurality of velocity curves is discussed in the following scientific article, which should be considered to be incorporated by reference: Bonnefous et al., Quantification of arterial flow using digital subtraction angiography, Med. Phys. 39 (10), October 2012. Additionally or alternatively, the velocity curves may be generated with the teaching of: Seifalian et al. A new algorithm for deriving pulsatile blood flow waveforms tested using simulated dynamic angiographic data, Neuroradiology 31:263-269, 1989, incorporated herein by reference.

Providing the frame rate is sufficient (typically 15 fps), and background neatly suppressed, the techniques to which the above cited references refer to can be applied to estimate velocity curves at each point. The integration operations are constrained with the vessel footprint and within coherent vessel labels. This can be perfectly automatised and does not require any manual guidance. This produces, for every semantically identified pixel of the image, a velocity curve along the observed cardiac cycles.

The characteristic extractor 150 extracts one or more characteristic from the one or more velocity curves according to the one or more locations. As an example, the characteristic extractor extracts the flow direction, the peak magnitude of the curve, a phasicity, and a curve characteristic for each location. As an example, the curve characteristic may be the upstroke characteristic and the peak magnitude may be a blood flow resistance indication. The upstroke characteristic means the shape, rate of rise and associated characteristic of the velocity curve as it ascends past zero i.e., as blood flow changes direction. In an embodiment, the one or more characteristic comprises a degree of stenosis at a specific location.

The diagnostic component 160 generates diagnostic data. In one embodiment the diagnostic data is data directly generated from the velocity curves. The diagnostic data is associated with a specific location and thus is generated from the one or more characteristics and the semantic angiogram data. The latter provides meaningful location information.

In a further embodiment, one or more diagnostic rules 180 are supplied to the system as an input and the diagnostic component 160 applies the diagnostic rules to determine if one or more condition may be present within the field of view. This is done by comparing the one or more characteristic to one or more reference characteristic, and by comparing the specific location to one or more reference locations. If a particular characteristic is found in a particular location it may be determined that a condition is present. Both the location and characteristics must match the reference location and values/ranges for the rule to be met. If the characteristic is present but in the wrong location, or if in a particular location the characteristic is not present, there is insufficient evidence that the condition is present. Optionally, the diagnostic rules 180 are provided as part of the system and not via a separate input. Optionally, the diagnostic rules 180 are obtained over a wired or wireless connection. In embodiments, the diagnostic rules may be periodically automatically, or may be updated whenever the diagnostic component requests an up-to-date set of rules.

The medical field is continually evolving with new insights being generated every year. Many of these insights can be formulated at diagnostic rules for use with the invention. An example of such insights and how they may be used to support a diagnosis can be found in the following paper, which should be considered to be incorporated by reference: Kim and al, Interpretation of peripheral arterial and venous Doppler waveforms: A consensus statement from the Society for Vascular Medicine and Society for Vascular Ultrasound, Vascular Medicine, 2020, Vol. 25(5) 484-506.

In an embodiment, the application of diagnostic rules comprises generating a characteristic value based on the characteristic at the specific location and comparing the characteristic value to one or more reference characteristic value or range.

In an embodiment, the diagnostic component 160 further assesses one or more patient characteristic and compares it to further reference characteristic in generating diagnostic data. This may comprise, for example, one or more of the medical history of the patient, the sex of the patient, the current medical condition of the patient or the current medication of the patient. Accordingly diagnostic data may be generated responsive to the assessment of a further patient characteristic.

In an embodiment, the diagnostic component 160 generates diagnostic data corresponding to the one or more locations by evaluating one or more characteristic corresponding to a different location to the specific location. For example, one or more characteristic of a nearby vessel may be evaluated to generate diagnostic data or to confirm a diagnostic indication.

For each vessel, current consensuses determine what normal and abnormal velocity curves are. This is based on the observation of the velocity curve descriptors, on the local anatomy (for instance presence of a stenosis), and possibly in relation with surrounding vessels. All this material may be gathered in diagnostic database that is addressed by the current anatomy configurations and extracted velocity descriptors. For each vessel, a statement indicating normality / abnormality can be issued with a proper description, making explicit on which grounds this was deduced.

The composite view generator 170 generates a composite view 190 from the semantic angiogram data and the data from the diagnostic component. In an embodiment, this comprises overlaying one or more characteristic from the one or more velocity curve on to the field of view, thus providing a composite diagnostic view that provides anatomical information and diagnostic data 192.

Thus, the diagnostic data is gathered in one or several views that will summarize the clinical vascular situation. Overview maps can be created that will indicate which part of the vessels are labelled as abnormal. Those maps can be defined by the user and can be interactive - for example, clicking on a point will open pop-ups with the measured velocity curves and descriptors, with the local vessel characteristics, and with the diagnostic rules involved, comparing the normal and the observed situation.

In an embodiment, the system 10 further comprises a user interface (not shown) and displays the composite view 190 on the user interface. In a further embodiment, the user interface is configured to receive an input to allow the user to modify or manipulate the composite view 190 on the user interface. For example, the diagnostic data 192 may be toggled on or off (i.e., set to visible or not visible). In a further embodiment, one or more of the one or more diagnostic rules 180 is modifiable or selectable by a user resulting in a modified diagnostic indication of whether a condition is present at the specific location.

In an alternative embodiment, the system is remote from the user interface. For example, the system may be located on a remote server, positioned away from where the user is working. The system generates a composite view 190 which is displayed on a screen adjacent to the user. The composite view 190 may be transferred to the screen by any known transfer protocol, for example by ethernet or over a wireless connection.

In an embodiment, the system 10 generates angiograms of the same vascular field before and after a medical treatment. Thus, given a pair of angiograms of the same vascular field before and after treatment, the system 10 could also propose an aggregated view to provide the physician with a sense on how the different descriptors evolved thanks to the treatment. In this setting, it would not be required to have a pixel perfect registration of the angiograms: the composer could leverage the semantic vessel segmentation and the label map by providing the averaged descriptors evolution on a given vessel or part of a vessel.

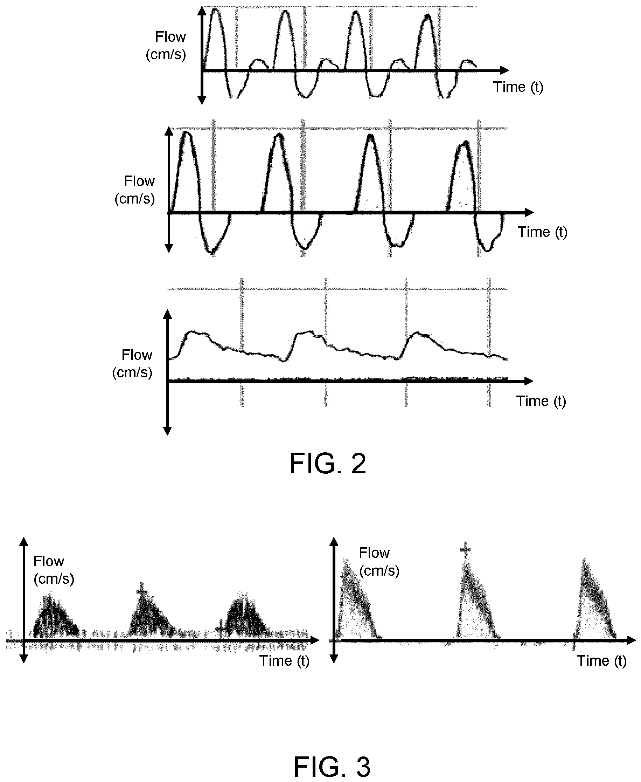

By way of further illustration and description the working of the invention will be described with reference to Figure 2 and Figure 3.

Figure 2 shows a plurality of velocity curves.

Figure 3 shows a comparison view of a velocity curve from a first position and a second position.

With reference to Figure 2, the three curves show how the velocity curves may indicate phasicity. The top curve indicates triphasic flow; the middle curve indicates biphasic flow; and the bottom curve indicates monophasic flow. Thus, phasicity may be determined from the velocity curves and may be a characteristic that is used in the subject invention.

Figure 3 shows a comparison view of a velocity curve from a first position and a second position. With reference to Figure 3, on the left is shown a velocity signal distal to an occlusion and on the right is shown a velocity signal proximal an occlusion. Accordingly, the location of a lesion can be determined, with respect to the positions at which the velocity curves are generated (e.g., distal or proximal to an occlusion).

As indicated in Figure 3 the location of a stenosis can be indicated by the shape of the waveform (damped vs rapid upstroke).

The shape of the velocity curve provides further insights which may be exploited in the subject invention. For example, resistance indication is described as follows: High-resistive curve indicated by sharp systolic upstroke, brisk downstroke; Low-resistive curve indicated prolonged downstroke in late systole with continuous forward flow throughout diastole without an end-of-systole notch. Such curves are monophasic; and intermediate-resistive curve: mixed high and low-resistive characteristics.

The upstroke may be used to support a diagnosis. The upstroke (beginning of systolic phase) can be rapid or prolonged. In the case of foot analysis, this can be referred to as Pedal Acceleration Time, which may be reliable indicator to evaluate lower limb arterial perfusion. In other words, the shape of the curve, in particular the upstroke, can be used to determine the presence of a condition or support a diagnosis.

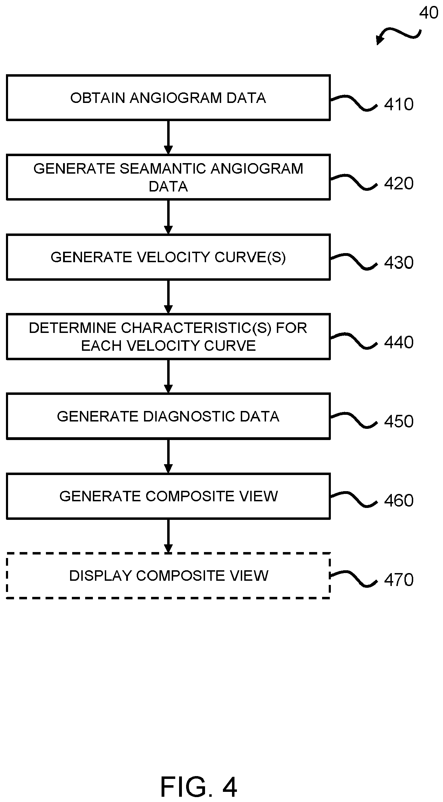

By way of further illustration and description, an exemplary method according to an embodiment will now be described with reference to Figure 4.

Figure 4 is a simplified flow diagram of a proposed vascular diagnosis method 40. The method 40 comprises several steps.

In an obtain angiogram data step 410, angiogram data is obtained. The data may be obtained directly by the system or may be provided to the system as an input. The angiogram data comprises a field of view.

In a generate semantic angiogram data step 420, semantic angiogram data is generated from the angiogram data.

In a generate velocity curve(s) step 430, one more velocity curves are generated corresponding to different positions in the field of view.

In a determine characteristic(s) step 440, one or more characteristics is generated from each of the one or more velocity curves.

In a generate diagnostic data step 450, diagnostic data is generated, the data corresponding to the one or more locations for which velocity curves have been generated. This diagnostic data is generated from the one or more characteristics and the semantic angiogram data.

In a generate composite view step 460, a composite view is generated.

In a display composite view step 470, the composite view is displayed. This step is optional.

The method steps may be further understood with reference to the description of the system and the operation of the functional components therein.

It should be understood that the present invention provides a method to generate vascular diagnosis imagery and/or data that may be used for vascular diagnosis. Thus, reference to a vascular diagnosis method should be understood to relate to a method of generating vascular diagnosis imagery and/or data that may be used for vascular diagnosis.

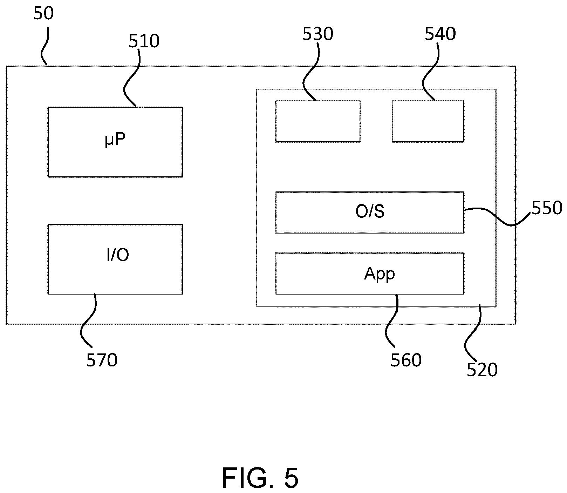

Figure 5 illustrates an example of a computer 50 within which one or more parts of an embodiment may be employed. Various operations discussed above may utilize the capabilities of the computer 50. For example, one or more parts of a system for providing a subject-specific user interface may be incorporated in any element, module, application, and/or component discussed herein. In this regard, it is to be understood that system functional blocks can run on a single computer or may be distributed over several computers and locations (e.g. connected via internet).

The computer 50 includes, but is not limited to, PCs, workstations, laptops, PDAs, palm devices, servers, storages, and the like. Generally, in terms of hardware architecture, the computer 50 may include one or more processors 510, memory 520, and one or more I/O devices 570 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 510 is a hardware device for executing software that can be stored in the memory 520. The processor 510 can be virtually any custom made or commercially available processor, a central processing unit (CPU), a digital signal processor (DSP), or an auxiliary processor among several processors associated with the computer 50, and the processor 510 may be a semiconductor based microprocessor (in the form of a microchip) or a microprocessor.

The memory 520 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 520 may incorporate electronic, magnetic, optical, and/or other types of storage media. Note that the memory 520 can have a distributed architecture, where various components are situated remote from one another, but can be accessed by the processor 510.

The software in the memory 520 may include one or more separate programs, each of which comprises an ordered listing of executable instructions for implementing logical functions. The software in the memory 520 includes a suitable operating system (O/S) 550, compiler 540, source code 530, and one or more applications 560 in accordance with exemplary embodiments. As illustrated, the application 560 comprises numerous functional components for implementing the features and operations of the exemplary embodiments. The application 560 of the computer 50 may represent various applications, computational units, logic, functional units, processes, operations, virtual entities, and/or modules in accordance with exemplary embodiments, but the application 560 is not meant to be a limitation.

The operating system 550 controls the execution of other computer programs, and provides scheduling, input-output control, file and data management, memory management, and communication control and related services. It is contemplated by the inventors that the application 560 for implementing exemplary embodiments may be applicable on all commercially available operating systems.

Application 560 may be a source program, executable program (object code), script, or any other entity comprising a set of instructions to be performed. When a source program, then the program is usually translated via a compiler (such as the compiler 540), assembler, interpreter, or the like, which may or may not be included within the memory 520, so as to operate properly in connection with the O/S 550. Furthermore, the application 560 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, .NET, and the like.

The I/O devices 570 may include input devices such as, for example but not limited to, a mouse, keyboard, scanner, microphone, camera, etc. Furthermore, the I/O devices 570 may also include output devices, for example but not limited to a printer, display, etc. Finally, the I/O devices 570 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 570 also include components for communicating over various networks, such as the Internet or intranet.

If the computer 50 is a PC, workstation, intelligent device or the like, the software in the memory 520 may further include a basic input output system (BIOS) (omitted for simplicity). The BIOS is a set of essential software routines that initialize and test hardware at startup, start the O/S 550, and support the transfer of data among the hardware devices. The BIOS is stored in some type of read-only-memory, such as ROM, PROM, EPROM, EEPROM or the like, so that the BIOS can be executed when the computer 50 is activated.

When the computer 50 is in operation, the processor 510 is configured to execute software stored within the memory 520, to communicate data to and from the memory 520, and to generally control operations of the computer 50 pursuant to the software. The application 560 and the O/S 550 are read, in whole or in part, by the processor 510, perhaps buffered within the processor 510, and then executed.

When the application 560 is implemented in software it should be noted that the application 560 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 560 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The present invention may be a system, a method, and/or a computer program product. The computer program product may include a computer readable storage medium (or media) having computer readable program instructions thereon for causing a processor to carry out aspects of the present invention.

The computer readable storage medium can be a tangible device that can retain and store instructions for use by an instruction execution device. The computer readable storage medium may be, for example, but is not limited to, an electronic storage device, a magnetic storage device, an optical storage device, an electromagnetic storage device, a semiconductor storage device, or any suitable combination of the foregoing. A non-exhaustive list of more specific examples of the computer readable storage medium includes the following: a portable computer diskette, a hard disk, a random access memory (RAM), a read-only memory (ROM), an erasable programmable read-only memory (EPROM or Flash memory), a static random access memory (SRAM), a portable compact disc read-only memory (CD-ROM), a digital versatile disk (DVD), a memory stick, a floppy disk, a mechanically encoded device such as punch-cards or raised structures in a groove having instructions recorded thereon, and any suitable combination of the foregoing. A computer readable storage medium, as used herein, is not to be construed as being transitory signals per se, such as radio waves or other freely propagating electromagnetic waves, electromagnetic waves propagating through a waveguide or other transmission media (e.g., light pulses passing through a fiber-optic cable), or electrical signals transmitted through a wire.

Computer readable program instructions described herein can be downloaded to respective computing/processing devices from a computer readable storage medium or to an external computer or external storage device via a network, for example, the Internet, a local area network, a wide area network and/or a wireless network. The network may comprise copper transmission cables, optical transmission fibers, wireless transmission, routers, firewalls, switches, gateway computers and/or edge servers. A network adapter card or network interface in each computing/processing device receives computer readable program instructions from the network and forwards the computer readable program instructions for storage in a computer readable storage medium within the respective computing/processing device.

Computer readable program instructions for carrying out operations of the present invention may be assembler instructions, instruction-set-architecture (ISA) instructions, machine instructions, machine dependent instructions, microcode, firmware instructions, state-setting data, or either source code or object code written in any combination of one or more programming languages, including an object oriented programming language such as Smalltalk, C++ or the like, and conventional procedural programming languages, such as the "C" programming language or similar programming languages. The computer readable program instructions may execute entirely on the user's computer, partly on the user's computer, as a stand-alone software package, partly on the user's computer and partly on a remote computer or entirely on the remote computer or server. In the latter scenario, the remote computer may be connected to the user's computer through any type of network, including a local area network (LAN) or a wide area network (WAN), or the connection may be made to an external computer (for example, through the Internet using an Internet Service Provider). In some embodiments, electronic circuitry including, for example, programmable logic circuitry, field-programmable gate arrays (FPGA), or programmable logic arrays (PLA) may execute the computer readable program instructions by utilizing state information of the computer readable program instructions to personalize the electronic circuitry, in order to perform aspects of the present invention.

Aspects of the present invention are described herein with reference to flowchart illustrations and/or block diagrams of methods, apparatus (systems), and computer program products according to embodiments of the invention. It will be understood that each block of the flowchart illustrations and/or block diagrams, and combinations of blocks in the flowchart illustrations and/or block diagrams, can be implemented by computer readable program instructions.

A single processor or other unit may fulfill the functions of several items recited in the claims.

A computer program may be stored/distributed on a suitable medium, such as an optical storage medium or a solid-state medium supplied together with or as part of other hardware, but may also be distributed in other forms, such as via the Internet or other wired or wireless telecommunication systems.

These computer readable program instructions may be provided to a processor of a general-purpose computer, special purpose computer, or other programmable data processing apparatus to produce a machine, such that the instructions, which execute via the processor of the computer or other programmable data processing apparatus, create means for implementing the functions/acts specified in the flowchart and/or block diagram block or blocks. These computer readable program instructions may also be stored in a computer readable storage medium that can direct a computer, a programmable data processing apparatus, and/or other devices to function in a particular manner, such that the computer readable storage medium having instructions stored therein comprises an article of manufacture including instructions which implement aspects of the function/act specified in the flowchart and/or block diagram block or blocks.

The computer readable program instructions may also be loaded onto a computer, other programmable data processing apparatus, or other device to cause a series of operational steps to be performed on the computer, other programmable apparatus or other device to produce a computer implemented process, such that the instructions which execute on the computer, other programmable apparatus, or other device implement the functions/acts specified in the flowchart and/or block diagram block or blocks.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

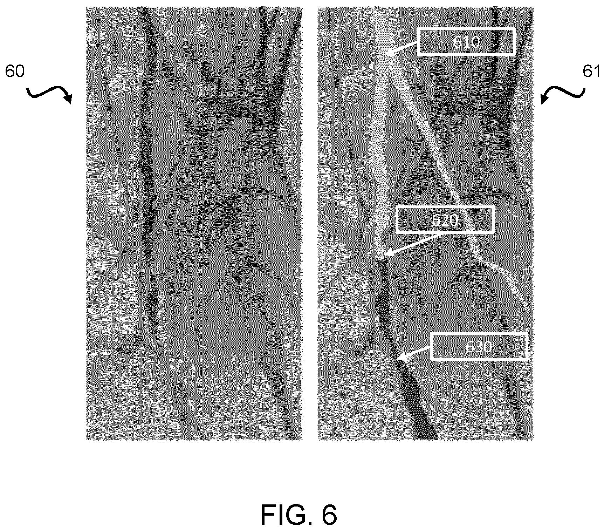

Figure 6 illustrates a possible diagnostic view 61 resulting from the invention, alongside unprocessed angiogram imagery 60. The field of view comprises the angiogram imagery and further may comprise one or more of the following: one or more marker or label 610, which may indicate information about a vessel or feature present in the field of view; one or more reference to diagnostic data 620 relating to one or more points in time, for example the diagnostic view may comprise an indication of an average or maximum flow value at a specific location; and a diagnostic indication 630. Furthermore, the diagnostic view 61 may comprise one or more of shading, colouring, highlighting and outlining, for example to highlight particular vessels or features, or to link aspects of the field of view together (for example, linking vasculature that is related in some manner, such as two different vessels that have characteristics that support a specific diagnostic indication 630).

The mere fact that certain measures are recited in mutually different dependent claims does not indicate that a combination of these measures cannot be used to advantage.

If the term "adapted to" is used in the claims or description, it is noted the term "adapted to" is intended to be equivalent to the term "configured to".

Any reference signs in the claims should not be construed as limiting the scope.

## Claims

1. A vascular diagnosis system, comprising
at least one processor, the at least one processor configured to:
obtain angiogram data of a patient's vascular system, said angiogram data comprising a field of view of a patient's vascular system;
process the angiogram data to generate a semantic angiogram data comprising information on the presence and position of anatomical features present in the field of view;
process the semantic angiogram data to determine one or more velocity curves according to one or more locations, wherein a velocity curve comprises data of blood velocity against time for a specific location in the field of view;
determine one or more characteristics from the one or more velocity curves according to the one or more locations;
generate diagnostic data corresponding to the one or more locations, from the one or more characteristics and the semantic angiogram data; and
generate a composite view comprising the diagnostic data.

2. The system according to claim 1, wherein the diagnostic data corresponding to the one or more locations is generated by applying one or more diagnostic rules at each of the specific locations of the one or more locations, wherein each of the one or more diagnostic rules comprises:
comparing the one or more characteristic to one or more reference characteristic; and
comparing the specific location to one or more reference locations,
to determine a diagnostic indication of whether a condition is present at the specific location.

3. The system according to claim 2, wherein comparing the one or more characteristic to one or more reference characteristic comprises generating a characteristic value based on the characteristic at the specific location and comparing the characteristic value to one or more reference characteristic value or range.

4. The system according to claim 2 or claim 3, wherein the processor is configured to obtain one or more further patient characteristic, and wherein
the diagnostic rule further comprises comparing the further patient characteristic to one or more further reference characteristic.

5. The system according to any of claims 2 to 4, wherein the one or more characteristic comprises a degree of stenosis at a specific location.

6. The system according to any of claims 2 to 5, wherein the generating diagnostic data corresponding to the one or more locations comprises evaluating one or more characteristic corresponding to a different location to the specific location.

7. The system according to any preceding claim, wherein one or more of the one or more diagnostic rules is modifiable or selectable by a user resulting in a modified diagnostic indication of whether a condition is present at the specific location.

8. The system according to any preceding claim, wherein the at least one processor is configured to generate:
a vessel segmentation map comprising information on which portions of the field of view comprise vessels; and
one or more labels, comprising an identification of a specific vessel present the field of view and an associated position of the specific vessel;
enabling the extraction of at least one semantic vessel from the vessel segmentation map, enabling the processing of the angiogram data to generate a semantic angiogram data comprising information on the presence and position of anatomical features present in the field of view.

9. The system according to any preceding claim, wherein the characteristic comprises one or more of a flow direction, a phasicity, or a curve characteristic.

10. The system according to claim 9, wherein the curve characteristic comprises one or more of a blood flow resistance indication or an upstroke characteristic.

11. The system according to any preceding claim, wherein the at least one processor is configured to process the angiogram data to compensate for a motion present when the angiogram data is generated, resulting in the generation of motion-compensated semantic angiogram data, and optionally wherein the motion present comprises one or more of a patient movement; an organ movement; and a table movement.

12. The system according to any preceding claims, further comprising a user interface, and wherein the at least one processor is configured to display the composite view on the display, and optionally wherein the user interface is configured to receive an input to allow the user to modify or manipulate the composite view on the user interface.

13. The system according to any preceding claim, wherein the one or more characteristic comprises the pulsatility of blood flow and the composite view comprises a blood pulsatility map corresponding to the field of view.

14. A computer-implemented method of vascular diagnosis, comprising the steps of:
obtaining angiogram data of a patient's vascular system, said angiogram data comprising a field of view of a patient's vascular system;
processing the angiogram data to generate a semantic angiogram data comprising information on the presence and position of anatomical features present in the field of view;
processing the semantic angiogram data to determine one or more velocity curves according to one or more locations, wherein a velocity curve comprises data of blood velocity against time for a specific location in the field of view;
determining one or more characteristic from the one or more velocity curves according to the one or more locations;
generating diagnostic data corresponding to the one or more locations, from the one or more characteristics and the semantic angiogram data; and
generating a composite view comprising the diagnostic data.

15. A computer program product comprising computer program code means which, when executed on a computing device having a processing system, cause the processing system to perform all of the steps of the method according to claim 14.
